Europäisches Patentamt

European Patent Office

Office européen des brevets

(11) Veröffentlichungsnummer: **0 103 185**
**A1**

(12) # EUROPÄISCHE PATENTANMELDUNG

(21) Anmeldenummer: 83108000.7

(51) Int. Cl.³: **A 61 B 5/02**

(22) Anmeldetag: **12.08.83**

(30) Priorität: **08.09.82 DE 3233245**

(43) Veröffentlichungstag der Anmeldung: **21.03.84**
**Patentblatt 84/12**

(84) Benannte Vertragsstaaten: **AT BE CH DE FR GB IT LI LU NL SE**

(71) Anmelder: **INTERMEDICAT GMBH, Gerliswilstrasse 74, CH-6020 Emmenbrücke (CH)**

(72) Erfinder: **Koch, Heinrich, Haarbreite 41, D-3436 Hess. Lichtenau/Walburg (DE)**

(74) Vertreter: **von Kreisler, Alek, Dipl.-Chem. et al, Deichmannhaus am Hauptbahnhof, D-5000 Köln 1 (DE)**

(54) Einrichtung zur Bestimmung des Herzzeitvolumens.

(57) Zur Bestimmung des Herzzeitvolumens nach der Thermodilutionsmethode wird eine gekühlte Kochsalzlösung als Indikator in das Blut eingespritzt. Anschließend wird über einen mit einem Katheter in das Herz eingeführten Thermistor (10) der Temperaturverlauf des Blut-Indikatorgemisches im rechten Vorhof des Herzens gemessen. Da der Thermistor (10) Bestandteil des Katheters ist, der bei jedem Patienten gewechselt wird, müssen kostengünstige Thermistoren benutzt werden, die jedoch hinsichtlich ihrer thermoelektrischen Kennlinie erhebliche Exemplarstreuungen aufweisen. Um einen exakten Temperaturwert zu erhalten, ist an dem Katheter ein Datenträger (26) angebracht, der Kennliniendaten des betreffenden Thermistor trägt. Diese Kennliniendaten werden von einem Kartenleser (25) gelesen und einer Speicher- und Umsetzschaltung (24) zugeführt. Anschließend wird der Speicher- und Umsetzschaltung (24) der Meßwert des Thermistors (10) zugeführt und unter Berücksichtigung der Kennliniendaten in den exakten Temperaturwert umgesetzt.

0103185

Einrichtung zur Bestimmung des Herzzeitvolumens

Die Erfindung betrifft eine Einrichtung zur Bestimmung des Herzzeitvolumens, mit einem Katheter, der
an seinem in das Herz einführbaren Ende ein temperaturabhängiges elektrisches Bauteil trägt, und mit einer
über Leitungen und eine Steckverbindung mit dem Bauteil verbundenen Auswerteschaltung zur Ermittlung des
Herzzeitvolumens in Abhängigkeit von dem an dem Bauteil
auftretenden Spannungsabfall.

Die Bestimmung der Pumpleistung des menschlichen
Herzens, im medizinischen Sprachgebrauch "Herzzeitvolumen" genannt, erfolgt üblicherweise mit Hilfe
sogenannter Indikatordilutionsverfahren. Hierzu wird
in den rechten Vorhof ein Indikator möglichst als
Bolus gespritzt und die Vermischung des Indikators
mit dem Blut, d.h. seine Konzentration z.B. in der
Pulmonalarterie als Funktion der Zeit gemessen. Aus
diesem Konzentrationsprofil und der Menge des eingespritzten Indikators läßt sich das Herzzeitvolumen

- 2 -

(abgekürzt HZV, Einheit: ℓ/min) nach einer von Steward und Hamilton abgeleiteten Formel berechnen.

Als Indikator kann ein physiologisch unbedenklicher Farbstoff verwendet werden (Dye-Dilution, DD); es wird allerdings immer gebräuchlicher, den (im Vergleich zum Blut) fehlenden Wärmeinhalt einer gekühlten physiologischen Kochsalzlösung auszunutzen (Thermodilution, TD). Dieses letztere Verfahren hat den Vorteil des geringeren meßtechnischen Aufwandes, da man als Sensor lediglich einen Thermistor oder ein anderes temperaturabhängiges elektrisches Bauteil an der Spitze eines mehrlumigen, auch zur Injektion des Indikators dienenden Katheters anbringen muß, der ausgehend von einer peripheren Vene bis in die Pulmonalarterie vorgeschoben wird. Dagegen erfordert die Dye-Dilution-Methode mindestens einen weiteren, meist arteriellen Zugang am Gefäßsystem, aus dem kontinuierlich Blut entnommen und in eine Küvette geleitet und die Farbstoffkonzentration dort mit Hilfe eines Spektrophotometers bestimmt wird.

Bei beiden Verfahren erfolgt die Auswertung dann üblicherweise mit Hilfe eines analog oder digital arbeitenden Computers.

Die Steward-Hamilton-Formel in der für das TD-Verfahren gültigen Fassung lautet

$$HZV = \frac{\text{const. } (T_{BO} - T_i) \cdot V_i}{\int_0^\infty (T_{BO} - T_{B(t)})\, dt}$$

0103185

- 3 -

wobei $T_{Bo}$ die normale Bluttemperatur, $T_i$ und $V_i$ Temperatur und Volumen der Indikatorlösung und $T_B$ (4) die (zeitabhängige) Temperatur des Blut-Indikatorgeschmisch in der Pulmonalarterie angeben. Die Konstante charakterisiert weitere Versuchsbedingungen wie z.B. das Volumen des zur Injektion verwandten Kanals im Katheter und die spezifische Wärme von Blut und Injektat.

Üblicherweise werden als Meßfühler für die Temperaturmessung des Blutes und des Blut-Indikator-Gemisches Thermistoren verwendet, d.h. der temperaturabhängige elektrische Widerstand des Meßfühlers wird mit Hilfe einer Wheatstone-Brücke und eines daran angeschlossenen Differenzverstärkers erfaßt und einem Rechner zugeführt, der die Umrechnung der gemessenen Widerstandswerte in Temperaturwerte vornimmt und die weitere Auswertung durchführt.Dabei dient ein an einen Differenzverstärker angeschlossene erste Meßbrücke, in die der Thermistor des Katheters geschaltet ist zur Bestimmung der Temperatur des Blutes bzw. des Blut-Indikator-Gemisches. An den anderen Eingang des Differenzverstärkers ist eine zweite Meßbrücke geschaltet, die ebenfalls einen Thermistor enthält und zur Bestimmung der Indikatortemperatur unmittelbar vor der Injektion benutzt wird. Der Thermistor der zweiten Meßbrücke ist Bestandteil des Gerätes, d.h. er wird bei aufeinanderfolgenden Einsätzen des Gerätes immer wieder verwendet, so daß eine Anpassung von Thermistor und Meßbrücke bereits werksseitig vorgenommen werden kann. Außerdem kann für die zweite Meßbrücke ein hochwertiger und teurer Thermistor benutzt werden.

- 4 -

Dagegen handelt es sich bei den Thermodilutionskathetern um medizinische Einmalartikel, die nach erfolgter Messung fortgeworfen werden sollten. Hier
müssen aus Gründen der Kostenersparnis preisgünstigere
Thermistoren, in der Regel aus Metalloxydkeramik,
eingesetzt werden. Man wird zwar für die Bestückung des
Katheters immer denselben Thermistortyp verwenden, auf
dessen durchschnittliche Kenndaten die Auswerteschaltung
eingestellt ist, jedoch können bei Thermistortypen der
genannten Art erhebliche Exemplarstreuungen auftreten,
.d.h. die tatsächliche Kennlinie eines Thermistors kann
um bis zu 20% von den Mittelwerten der Serie abweichen.
Diese Streuung wird zwar von den Herstellern der Katheter
durch Selektion auf bis zu 5% eingeengt, jedoch ist dies
noch nicht ausreichend, da bei einer Toleranzbreite
von 5% die Abweichungen des gemessenen Herzzeitvolumens
vom tatsächlichen Herzzeitvolumen bis zu 10% betragen
können. Der Anwender hat keine Möglichkeit diesen
Fehler zu korrigieren. Ein weiterer Fehler entsteht dadurch, daß die Auswerteschaltung die Kennlinie des
Thermistors im interessierenden Temperaturbereich als
linear annimmt, was aber nicht der Fall ist.

Der Erfindung liegt die Aufgabe zugrunde, eine Einrichtung der eingangs genannten Art zu schaffen, mit
der der Einfluß der Exemplarstreuungen der temperaturabhängigen elektrischen Bauteile auf das Meßergebnis
ausgeschaltet wird, ohne der Auswerteschaltung manuell
Korrekturdaten eingeben zu müssen.

- 5 -

Zur Lösung dieser Aufgabe ist erfindungsgemäß vorgesehen, daß an dem Katheter ein maschinenlesbarer Datenträger angebracht ist, der Kennliniendaten über die thermoelektrische Kennlinie des temperaturabhängigen Bauteils des Katheters enthält, und daß die Auswerteschaltung eine Speicher- und Umsetzschaltung enthält, die über ein Lesegerät die Kennliniendaten empfängt und die ein ihr anschließend zugeführtes Spannungssignal in Abhängigkeit von den Kennliniendaten in einen Temperaturwert umsetzt.

Hierbei besteht die Möglichkeit, die Kennliniendaten bereits bei der Herstellung des Katheters auf dem Datenträger anzubringen. Unmittelbar vor der Messung werden die Kennliniendaten durch Ablesen des Datenträgers von dem Lesegerät in die Speicher- und Umsetzschaltung eingegeben, wo sie gespeichert werden. Die Kennliniendaten des Thermistors werden vom Hersteller durch Ausmessen der Thermistorkennlinie ermittelt und auf den Datenträger aufgezeichnet. Da die anschließende Übertragung in die Auswerteschaltung maschinell erfolgt, sind Einstell- und Übertragungsfehler ausgeschlossen. Zur Vorbereitung der Messung des Herzzeitvolumens ist es lediglich erforderlich, außer den ohnehin vorzunehmenden elektrischen Anschlußarbeiten den Datenträger an dem Lesekopf eines mit der Auswerteschaltung verbundenen Lesegerätes entlangzuführen. Auf diese Weise ist es möglich, nicht nur einige wenige charakteristische Parameter der Thermistorkennlinie einzugeben, sondern der Auswerteschaltung die gesamte Kennlinie mitzuteilen, beispielsweise in Form von Geradenabschnitt nach der Methode der linearen Aproximation oder in Form zahlreicher

- 6 -

Einzeldaten. Zusätzlich können auch noch andere katheterspezifische Daten, die für die Auswertung wichtig sind, wie z.B. das Kanalvolumen, auf dem Datenträger vermerkt sein, um die Konstante const. der oben angegebenen Steward-Hamilton-Formel entsprechend zu variieren.

Als Datenträger kann beispielsweise eine Magnetkarte eingesetzt werden. Wichtig ist, daß der Datenträger unverlierbar am Katheter angebracht ist, so daß seine Zuordnung zu dem betreffenden Thermistor sichergestellt ist.

Gemäß einer vorteilhaften Weiterbildung der Erfindung ist vorgesehen, daß der Datenträger an einem die Leitungen des temperaturabhängigen Bauteils mit der Auswerteschaltung verbindenden Stecker derart angebracht ist, daß sein Inhalt beim Einstecken des Steckers in den zugehörigen Gegenstecker am Gehäuse der Auswerteschaltung von dem Lesegerät gelesen wird. Dies hat den Vorteil, daß es nicht einmal erforderlich ist, den Datenträger in das Lesegerät einzuführen, sondern daß der Datenträger beim Herstellen der elektrischen Verbindung selbsttätig abgelesen wird. Als Datenträger können natürlich auch optisch oder mechanisch abtastbare Systeme Verwendung finden.

Im folgenden wird unter Bezugnahme auf die Zeichnungen ein Ausführungsbeispiel der Erfindung näher erläutert.

Es zeigen:

Fig. 1     ein Blockschaltbild des elektrischen Teils
           der Einrichtung zur Bestimmung des Herzzeitvolumens,

Fig. 2     eine perspektivische Darstellung eines
           Steckers mit Datenträger, und

Fig. 3     eine Ansicht des Katheters, teilweise ge-
           schnitten.

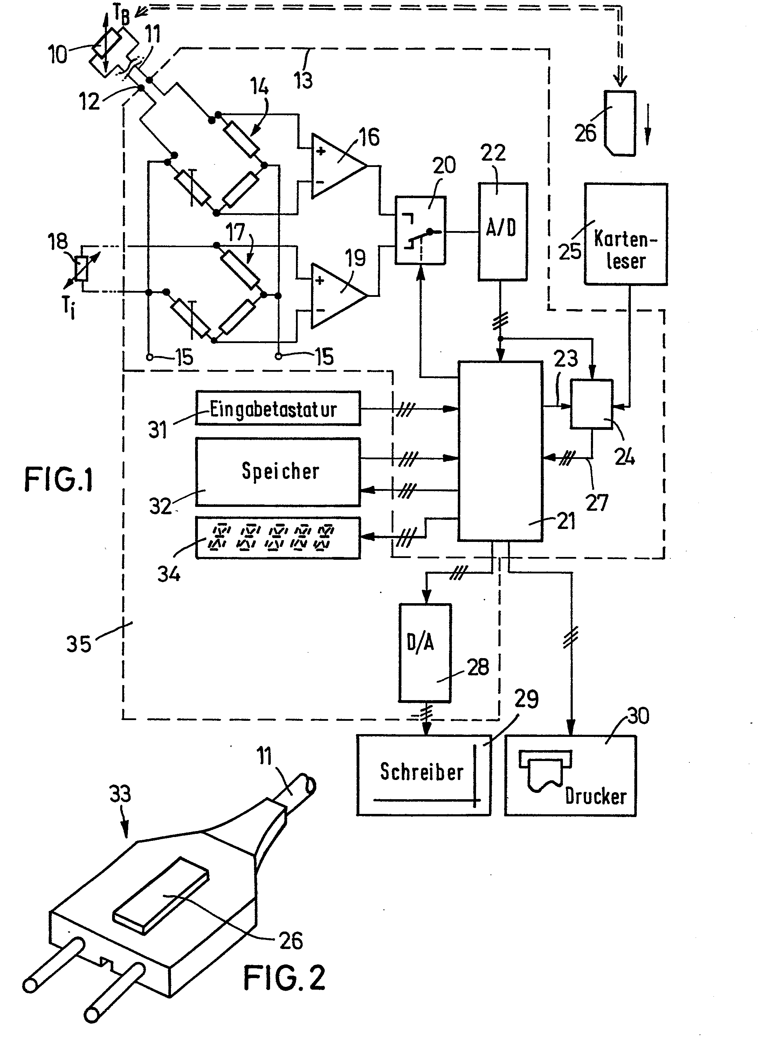

Gemäß Fig. 1 ist ein Thermistor 10, der Bestandteil
eines Thermodilutionskatheters ist, mit seinen Anschlußleitungen 11 über eine Steckverbindung   12 mit
der Auswerteschaltung 13 verbunden. Die Auswerteschaltung 13 enthält eine erste Meßbrücke 14, in
deren einen Meßzweig der Thermistor 10 geschaltet ist.
Die Meßbrücke 14 ist über die Klemmen 15 an eine Versorgungsspannung angeschlossen. Die Meßpunkte der
Meßbrücke 14 sind mit den beiden Eingängen eines
Differenzverstärkers 16 verbunden.

Eine zweite Meßbrücke 17 enthält als einen der
Brückenwiderstände einen zweiten Thermistor 18, der
sich außerhalb des Gerätes befindet und zur Messung
der Indikatortemperatur dient. Dieser Thermistor 18
wird normalerweise nicht ausgewechselt, da er mit dem
Körper des Patienten nicht in Berührung kommt. Die zweite
Meßbrücke 17, die ebenfalls an die Versorgungsspannung
15 angeschlossen ist, ist mit ihrem Meßzweig an die
beiden Eingänge eines Differenzverstärkers 19 angeschlossen. Die Ausgänge der beiden Differenzverstärker 16 und 19 sind mit einem elektronischen
Schalter 20 verbunden, der von einem Rechner 21 gesteuert ist. Auf diese Weise wird einem dem Schalter 20
nachgeschalteten Analog/Digital-Umsetzer in Abhängigkeit von der Stellung des Schalters 20 entweder das

- 8 -

Signal des Differenzverstärkers 16 oder das Signal des Differenzverstärkers (19) zugeführt. Das Ausgangssignal des Analog/Digital-Umsetzers 22 wird auf ein Steuersignal des Rechners 21 an Leitung 23 einer Speicher- und Umsetzschaltung 24 zugeführt, deren Funktion nachfolgend noch erläutert wird.

In die Speicher- und Umsetzschaltung 24 werden über einen Kartenleser 25 die von einem Datenträger 26 .abgelesenen Kennliniendaten des Thermistors 10 zugeführt. Diese Kennliniendaten können entweder aus ausgewählten Koordinatenwerten der thermoelektrischen Kennlinie des Thermistors 10 bestehen oder aus Daten, die den Kennlinienverlauf in anderer Weise repräsentieren. Der Speicher- und Umsetzschaltung wird auf diese Weise unter Steuerung durch den Rechner 21 zunächst die thermoelektrische Kennlinie des betreffenden Thermistors 10 eingegeben. Anschließend wird der Speicher- und Umsetzschaltung 24 das Ausgangssignal des Differenzverstärkers 16 in digitaler Form zugeführt. Die Speicher- und Umsetzschaltung 24 erzeugt daraufhin über die Leitungen 27 ein Temperatursignal, das der Temperatur des Thermistors 10 unter Berücksichtigung der individuellen Kennlinie dieses Thermistors entspricht und das dem Rechner 21 zur Verarbeitung zugeführt wird.

Das Ausgangssignal des Differenzverstärkers 19 wird nach Umwandlung durch den Analog/Digital-Umsetzer in digitaler Form direkt dem Rechner 21 zugeführt, also nicht über die Speicher- und Umsetzschaltung 24. Der Rechner 21 errechnet aus der vom Thermistor 18 gemessenen Temperatur $T_i$ der Indikatorlösung und der vom Thermistor 10 gemessenen Temperatur $T_B$ des Blut-Indikatorgemisches nach der oben angegebenen Steward-Hamilton-Formel das Herzzeitvolumen und liefert einen entsprechenden Wert an einen Drucker 30 bzw. über einen Digital/Analog-Umsetzer 28 an einen Kurvenschreiber 29.

An den Rechner 21 sind außerdem eine Eingabetastatur 31, ein externer Speicher 32 und eine Ziffernanzeigeeinrichtung 34 angeschlossen. Diese Komponenten sind zusammen mit der Auswerteschaltung 13 und dem Kartenleser 25 in einem Gerät 35 zusammengefaßt.

Der Datenträger 26 ist unverlierbar an dem Katheter, dessen Bestandteil der Thermistor 10 ist, befestigt, was in Fig. 1 durch die gestrichelte Doppellinie angedeutet ist. Er wird nach dem Herstellen der Steckverbindung 12 an dem Lesekopf des Kartenlesers 25 entlanggeführt. Der (nicht dargestellte) Lesekopf kann aus einem bewegbaren Stift bestehen, der über ein flexibles Kabel mit dem Gehäuse des Kartenlesers 25 verbunden ist und an dem Datenträger 26 entlangbewegt wird. In diesem Fall braucht der Datenträger an dem Katheter keine größere Bewegungsfreiheit zu haben.

Bei dem beschriebenen Ausführungsbeispiel ist die Speicher- und Umsetzschaltung 24 als separate Baugruppe außerhalb des Rechners 21 dargestellt. Ihre Funktion kann aber auch bei entsprechender Programmierung unmittelbar vom Rechner 21 ausgeführt werden.

Fig. 2 zeigt einen Flachstecker 33, der das Ende eines zu dem Thermistor 10 führenden Kabels 11 bildet und der in Verbindung mit einem (nicht dargestellten)

- 10 -

Gegenstecker am Gehäuse der Auswerteschaltung 13 die
Steckverbindung 12 bildet. Auf die eine Breitseite des
Flachsteckers 33 ist der Datenträger 26 aufgeklebt.
Der Lesekopf des Lesegerätes 25 befindet sich am Gehäuse
der Auswerteschaltung 13 an der Stelle, an der beim Einschieben des Steckers 33 in die entsprechende Steckerfassung der Datenträger 26 entlangbewegt wird. Auf diese
Weise werden die auf dem Datenträger 26 enthaltenen
Kennliniendaten und ggf. andere charakteristische Daten
des betreffenden Katheters beim Einschieben des Steckers
33 in den entsprechenden Gegenstecker bzw. beim Herstellen der Kontaktverbindung 12 selbsttätig gelesen, so
daß zum Einlesen der Katheter- bzw. Thermistordaten in
die Speicher- und Umsetzschaltung 24 keine zusätzlichen
Handgriffe erforderlich sind.

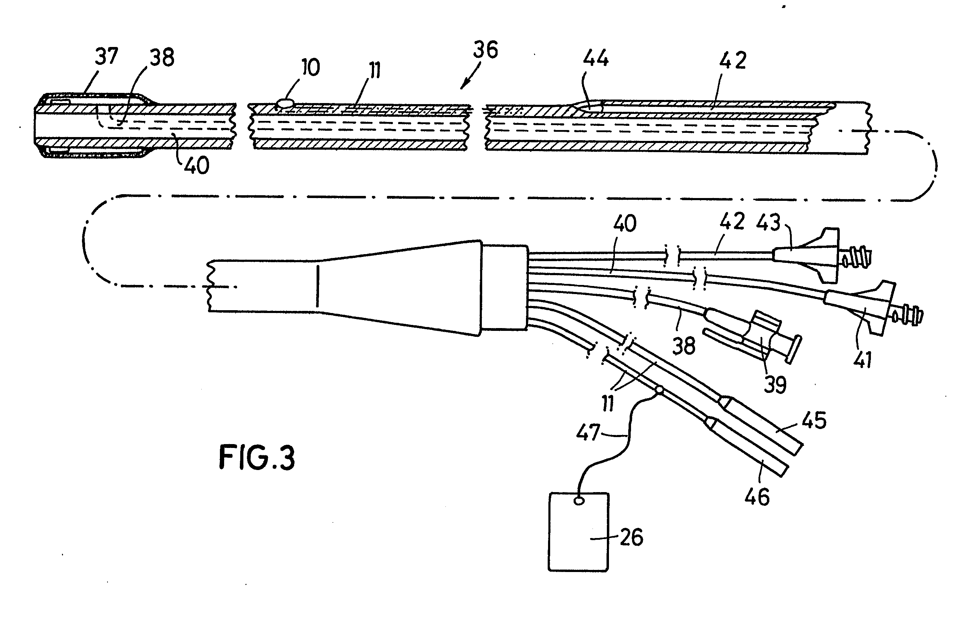

Fig. 3 zeigt eine Darstellung des Katheters 36, der den
Thermistor 10 enthält und für die Durchführung der
Messung des Herzzeitvolumens benutzt wird. Der Katheter
trägt an seinem patientenseitigen Ende einen kleinen
Ballon 37, der über eine sich durch den Katheter erstreckende Leitung 38 aufgeblasen werden kann. Die
Leitung 38 ist an dem patientenfernen Ende mit einem Anschlußstück 39 versehen, das einen Absperrhahn aufweist.
Der aufgeblasene Ballon erleichtert die Passage der
Katheterspitze durch das Herz in die Pulmonalarterie durch
sog. Einschwemmtechnik. Er dient ferner zum Verschließen
eines Nebenastes der Pulmonalarterie zu Diagnosezwecken.

Der Kather 36 weist ferner einen durchgehenden Kanal 40
auf, der sich über die gesamte Länge des Katheters erstreckt und an seinem patientenfernen Ende mit einem Anschlußstück 41 versehen ist. Zur Messung des Herzzeitvolumens dient ein weiterer Kanal 42, der an
seinem patientenfernen Ende ebenfalls ein Anschlußstück 43 aufweist. Dieser Kanal 42 endet in einer
seitlichen Öffnung 44 des Katheters, die sich etwa
30 cm hinter dem Ballon 37 befindet. Durch den Kanal
42 wird der Indikator eingespritzt.

Der Thermistor 10 befindet sich etwa 4 cm hinter der
Katheterspitze. Von ihm erstrecken sich die elektrischen
Leitungen 11 bis zum patientenfernen Katheterende, wo
sie mit Anschlußsteckern 45,46 versehen sind. Der Datenträger 26 ist beispielsweise mit einer Schnur 47 unverlierbar an einer der Leitungen 11 angebracht. Er ist
so ausgebildet, daß er auch bei einer Resterilisierung
des Katheters 36 keinen Schaden nimmt.

- 12 -

Ansprüche

1. Einrichtung zur Bestimmung des
   mit einem Katheter, der an seinem in das Herz einführbaren Ende ein temperaturabhängiges elektrisches Bauteil trägt,und mit einer über Leitungen und eine
   Steckverbindung mit dem Bauteil verbundenen Auswerteschaltung zur Ermittlung des Herzzeitvolumens in Abhängigkeit von dem an dem Bauteil auftretenden Spannungsabfall, d a d u r c h   g e k e n n z e i c h n e t ,
   daß an dem Katheter ein maschinenlesbarer Datenträger
   .(26) angebracht ist, der Kennliniendaten über die
   thermoelektrische Kennlinie des temperaturabhängigen
   Bauteils (10) des betreffenden Katheters enthält, und
   daß die Auswerteschaltung (13) eine Speicher- und
   Umsetzschaltung (24) enthält, die über ein Lesegerät
   (25) die Kennliniendaten empfängt und die ein ihr anschließend zugeführtes Meßsignal in Abhängigkeit von
   den Kennliniendaten in einen Temperaturwert umsetzt.

2. Einrichtung nach Anspruch 1, dadurch gekennzeichnet,
   daß der Datenträger (26) an einem die Leitungen (11)
   des Bauteils (10) mit der Auswerteschaltung (13) verbindenden Stecker (33) derart angebracht ist, daß sein
   Inhalt beim Einstecken des Steckers (33) in den zugehörigen Gegenstecker .am Gehäuse der Auswerteschaltung
   (13) von dem Lesegerät (25) gelesen wird.

$T_B$

10   11

12   13

14

16

+
−

20

22

A/D

26

25   Karten-
     leser

18

17

19

+
−

$T_i$

15   15

31   Eingabetastatur

23

21

24

27

32   Speicher

34

35

FIG.1

D/A

28

29   30

Schreiber

Drucker

33   11

26

FIG.2

FIG.3

0103185

## EINSCHLÄGIGE DOKUMENTE

EP 83108000.7

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | DE - A1 - 2 843 492 (OBERMAJER) -- | | A 61 B 5/02 |
| A | US - A - 4 240 441 (KHALIL) -- | | |
| A | US - A - 4 236 527 (NEWBOWER) -- | | |
| A | AT - B - 314 878 (SIEMENS) <br> * Seite 2, Zeilen 43-57; Fig. 2 * -- | 1 | |
| A | DE - A1 - 2 844 307 (MALLORY) <br> * Seite 5, Abs. 2 - Seite 6, Abs. 1; Fig. 1-3 * -- | 1 | |
| A | DE - B2 - 2 746 935 (KUKE) <br> * Spalte 2, Zeilen 39-67 * -- | 1 | RECHERCHIERTE SACHGEBIETE (Int. Cl. ³) |
| A | DE - A1 - 2 532 885 (WIRTH) <br> * Patentansprüche 1,6-9 * -- | 1 | A 61 B <br> G 01 K <br> H 01 R <br> G 06 F |
| A | GB - A - 2 047 013 (HOCHIKI) <br> * Seite 1, Zeilen 52-65 * -- | 1 | |
| A | US - A - 3 672 752 (YOUNG) <br> * Zusammenfassung; Spalte 1, Zeilen 28-37; Spalte 2, Zeilen 21-61 * -- | 1 | |

Der vorliegende Recherchenbericht wurde für alle Patentansprüche erstellt.

| Recherchenort | Abschlußdatum der Recherche | Prüfer |
|---|---|---|
| WIEN | 23-11-1983 | NEGWER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

| | Europäisches Patentamt | **EUROPÄISCHER RECHERCHENBERICHT** | Nummer der Anmeldung 0103185 |
|---|---|---|---|

EP 83108000.7

| **EINSCHLÄGIGE DOKUMENTE** | | | **KLASSIFIKATION DER ANMELDUNG (Int. Cl.³)** |
|---|---|---|---|
| Kategorie | Kennzeichnung des Dokuments mit Angabe, soweit erforderlich, der Maßgeblichen Teile | betrifft Anspruch | |
| A | PATENT ABSTRACTS OF JAPAN, unexamined applications, Section P, Band 6, Nr. 183, 18. September 1982 THE PATENT OFFICE JAPANESE GOVERNMENT, Seite 27 P 143 * Kokai-No. 57-96 325 (OLYMPUS) ---- | 1 | |
| | | | **RECHERCHIERTE SACHGEBIETE (Int. Cl.³)** |

EPA Form 1503.2   06.78